# NEW EUROPEAN PATENT SPECIFICATION

(11) **EP 0 342 034 B2**
(45) Date of publication and mention of the opposition decision: **20.08.1997**
(45) Mention of the grant of the patent: 27.10.1993
(21) Application number: 89304780.3
(22) Date of filing: 11.05.1989
(51) Int. Cl.: A61K 7/13

(54) **Process for dyeing hair by the sequential treatment with metal ion containing composition and dye composition containing 5,6-dihydroxyindole-2-carboxylic acid**
Verfahren zur Haarfärbung durch die aufeinanderfolgende Applikation einer Metallionen enthaltenden Zusammensetzung und einer eine 5,6-dihydroxy-2-karbonsäure enthaltenden Zusammensetzung
Procédé pour la décoloration des cheveux par le traitement séquentiel avec une composition contenant des ions de métaux et une composition contenant de l'acide 5,6-dihydroxyindole-2-carboxylique

(30) Priority: 12.05.1988 US 193389
(43) Date of publication of application: 15.11.1989
(73) Proprietor: Bristol-Myers Squibb Company, New York, N.Y. 10154 (US)
(72) Inventor: Schultz, Thomas Matthew, Highland Mills, N.Y. 10930 (US); Wolfram, Leszek J., Stamford, CT. 06902 (US); Brown, Keith C., New Caanan, CT 06840 (US); Prota, Giuseppe, Napoli (IT)
(74) Representative: Baillie, Iain Cameron

(56) References cited:
- EP-A- 0 271 186
- WO-A-88/01162
- DE-B- 1 083 505
- FR-A- 1 439 307
- GB-A- 2 132 642
- GB-A- 2 187 456
- JP-A-53 130 443
- PATENT ABSTRACTS OF JAPAN vol. 13, no. 562 (C-665)(3910) 13 December 1989, & JP-A-01 233210 (KAO CORP.) 19 September 1989,
- Mc Graw Hill dictionary of scientific and technical terms., 4th Ed., p. 17
- W. Montagna et al, Black skin, structure and function, 1993, pp. 84-85

## Description

### Background of Invention

This invention relates to processes for dyeing human hair and particularly for dyeing hair on the human head. More particularly, it concerns the use of 5,6-dihydroxyindole-2-carboxylic acid (sometimes referred to herein as DHICA) or specified derivative thereof in such processes.

Efforts have been made in the past to provide a hair dyeing process that mirrors the formation of hairs' natural pigment melanin. These efforts relied in general upon treatment of hair with a recognized intermediate of the melanin pathway--5,6-dihydroxyindole--DHI.

GB Publication No. 2 187 456 A discloses dying compositions for keratinous fibres based on indole derivatives that do not include the specified dihydroxy indole carboxylic acid derivatives of the subject invention.

EP-A-0 271 186 of Repligen Corporation discloses hydroxy indoles and their use in dying. Monohydroxy indoles and UV light or chemical oxidants such as sodium periodate are used, simultaneously or sequentially to dye hair or skin, but there is no disclosure of use of the dihydroxy indole carboxylic acids of the subject invention in such processes.

While the melanin formation can be successfully achieved by this approach, the results have left much to be desired. Firstly, DHI is a highly unstable compound and this presents considerable difficulty in product formulation. Secondly, the colors obtained on hair with DHI tends to be achromatic and thus require separate treatments to attain a broader range of shades.

Indeed color modulation of dihydroxyindole dyed hair has been taught to require either a pretreatment of the hair with a color modifier such as a metal ion, or iodide or a reducing agent prior to dye application. Alternatively, an oxidative posttreatment can be effectively used to the hair. Moreover, precursors such as 5,6-dihydroxyindole require a complicated synthesis which makes the use of this material expensive and often precludes its commercial use. The present invention eliminates the aforesaid disadvantages.

### The Invention

It has now been unexpectedly found that DHICA or specified derivatives thereof may be employed to dye human hair with soft shades of brown that emulate the naturally occurring melanin type pigments. Such dyeings use a process which involves sequentially treating hair with a transition metal ion containing composition, and with a hair dye composition containing DHICA, or specified derivatives thereof. Hair dyed in accordance with the process of the present invention has a natural lustre and sheen without a coated feel. Moreover the dyeouts have proven to have good light-and wet-fastness; that is to say, the dyeings obtained are photo- and shampooing-stable.

As used herein the term "sequentially treating" means that the metal ion containing composition and the DHICA or DHICA derivative containing compositions are applied one after the other without specifying which is to be applied first. In accordance with the present invention either order of application may be used; that is, the metal ion-containing compositions may be applied first followed by the application of the DHICA-containing or DHICA derivative containing compositions, or vice versa.

### Prior Art

There is ample patent history on the use of synthetic organic compounds which purport to mimic the natural coloring process in man. In the 1950's Raper and Mason (R.A. Nicolaus, "Melanins", in Chemistry of Natural Compounds, E. Lederer, ed., Herman Publishers, Paris, 1970), detailed a mechanism which accounted for the transformation of L-DOPA (3,4-dihydroxyphenylanine) to eumelanin, the primary coloring agent in man. The material was formed by oxidation of L-DOPA to DOPACHROME (a reddish material in solution) followed by conversion to either 5,6-dihydroxyindole (DHI) or 5,6-dihydroxyindole-2-carboxylic acid (DHICA). Subsequent oxidation of either DHI or DHICA ultimately yields the eumelanin. The entire process is regulated in-vivo by the enzyme tyrosinase.

It has been revealed recently that the Raper-Mason scheme for eumelanogenesis is incomplete and that a crucial component has been omitted. It now is apparent that the biological transformation of L-DOPA to DHI is not singular and it involves, rather, the additional formation of 5,6-dihydroxyindole -2-carboxylic acid, DHICA (S. Ito, Biochim. Biophys. Acta, 883, 155-161 [1986]). Indeed it was proposed by Swan in the 1970's that natural eumelanin was a mixed polymer incorporating DHI, DHICA, and L-DOPA (G.A. Swan, Zeitschrifte Natur. d. Org. Chem., "The Chemistry, Biochemistry, and Structure of Melanins", 152-186; 1976).

Several patents teach that the above process is mimicked by reaction of L-DOPA or tyrosine with an animal or vegetable tyrosinase and will dye hair a permanent black color (US 2539202, US 2875769, US 3993436, and JP 54110337). In addition, certain catalysts have been found effective in accelerating the dyeing of human hair: JP 53133641, JP 50130443, JP 8056159, US 4390341, US 4453941, EP 1610773A, and SU 566895 all teach that when L-DOPA and its derivatives are contacted with a metal-ion salt the reaction to dye hair brown or black will be accomplished much more readily. Several patents also teach that hair may be dyed using DHI either in conjunction with or serially applied with an oxidizing agent: US 2934396, DE 1083505, US 3194734, US 4208183, DE 2820193, and NL 830415/A. One of the major deficiencies in using DHI has been that the material is highly reactive and requires the utmost care both in its preparation and in its use, for it will spontaneously react to form granular materials which are ineffective in coloring hair.

The other drawback in DHI coloring systems, important from the color aesthetes, is the very limited range of hues than can be obtained. The dyeouts achieved via DHI use are primarily achromatic with the color intensity varying between black and grey, typically lacking warm brown or auburn tones without the addition of certain non-melanogenic materials.

In light of this, it was surprising to find that DHICA or specified DHICA derivatives effectively dye human hair. DHICA is a naturally occurring material that can now be used to dye hair soft shades of brown. The hair has a natural lustre and sheen without the coated feeling typical of hair dyed with conventional permanent hair colorants.

It has thus been very surprising to find that DHICA or the specified derivative can be effectively employed either alone or in mixture with DHI to provide a wide range of hair colors displaying a range of brown tonalities. Another advantage of DHICA is its ease of preparation (one step synthesis; H. Sobotha & J. Austin; J. Am. Chem., Soc.; 73, p 3077); and chemical stability during storage. We find that hair dyed with DHICA-containing compositions retains its luster and sheen without the raspy feel frequently observed in the case of conventional hair colorants.

### Detailed Description of Invention

The DHICA or specified DHICA deriative containing hair dye compositions disclosed herein may take any of a variety of forms. They may be made up into, e.g., gels, lotions, creams, aerosol compositions or the like or into simple emulsions, suspensions or solutions. One form that has been found to be useful is a solution of DHICA in a solvent system that is preferably an aqueous-organic solvent system. In such solutions, the organic solvent, which is preferably an alcohol, constitutes from 1% to 99% by volume of the solvent system, the balance generally being of water. In the preferred forms the organic solvent will constitute between 5% to 30% by volume of the solvent system and the water will comprise between 95% to 70% also on a volume basis.

A variety of organic solvents may be employed in the solvent system of the DHICA or specified DHICA derivative dye compositions disclosed herein. As indicated above alcoholic solvents have been found to be quite useful, especially those containing C₁ to C₆ alkanols and C₁ to C₆ ethers of such alkanols. By way of illustrating the solvents that are useful for the present purposes mention may be made of the following: ethanol, n-propanol, iso-propanol, ethoxyethanol, etc.

The quantity of DHICA or specified DHICA derivative that will be contained in the hair dye compositions disclosed herein may vary over a specified range depending on the results desired and the other ingredients contained in the composition, including other hair coloring agents. All that is required is that an effective hair dyeing quantity of DHICA be incorporated in the hair dye compositions. This specified range will fall within the range of from 0.001% to 10.0% by weight of DHICA or specified DHICA derivative based on the total weight of the hair dye composition, with the preferred concentration being from 0.01% to 5% on the same weight basis. Optimally, the DHICA or specified DHICA derivative concentration is about 1% by weight on the same weight basis.

This invention as foreshadowed above is not limited to DHICA proper. Such derivatives as the alkyl, aryl, or benzyl esters may be similarly employed (Structure I) to provide a source of the DHICA moiety during the hair dyeing process. Additionally N-substituted derivatives I, (R₁ = alkyl, aryl, or benzyl) may also be useful. It would be expected that such analogies of DHICA dye hair similarly but different from those taught for DHI and N-methyl-DHI. Indeed in the use of these latter two compounds color modulation of the dyeouts to light browns must be effected by incorporating into the process a bleaching agent as described in US 3,194,734. It is apparent that N-substitution of the indole only slightly changes the shade and addition of other agents is required to alter the tonal quality of the dyeout. 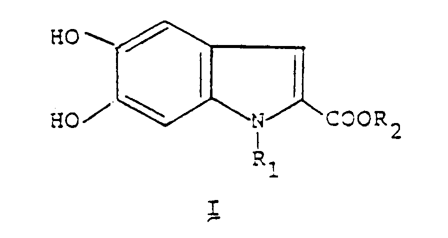
- R₁, R₂ =: H, alkyl, aryl, or benzyl

The pH of the DHICA- or specified DHICA derivative containing hair dye compositions can also vary over a range depending on the other ingredients in the composition and the results desired. Usually the pH of the composition will be from 3 to 10, the optimum pH being from 3 to 8.

It is sometimes advantageous to use a dihydroxindole (DHI) in admixture with DHICA or the specified DHICA derivative in the hair dye process of this invention. this provides the possibility of obtaining a wider range of colors on the hair. When a dihydroxyindole is employed it may vary in concentration depending on the quantity of the other ingredients contained in the composition and/or the results described. Generally, it will be present in the range from 0.01% to 4.0% by weight based on the total weight of the hair dye composition, with the preferred range being from 0.5% to 2.0% on the same weight bases.

A variety of dihydroxyindoles may be used for this purpose. By way of example mention may be made of dihydroxyindole (DHI), and N-methyldihydroxyindole (CH₃NDHI). Generally, N-substitutued DHI's in which the substituents are C₁ to C₆ alkyl groups in substituted or unsubstituted aryl groups are preferred.

Any of a number of adjuvants may also be added to the DHICA hair dye compositions disclosed herein. These may be added to facilitate the application of the dye composition to the hair, to improve the chemical or physical stability of the composition, to modify the color obtained in the dyeout or to improve the organoleptic properties of the composition. To these ends the DHICA hair dye composition may contain surfactants, foaming agents, metal scavengers, antioxidants, preservatives, auxiliary hair coloring agents, thickening agents, perfumes, product coloring agents, and other solvents.

The metal ion containing compositions used in this invention may contain any of a wide variety of metal ions which will be ions of a transition metal. These are chosen so as to provide a deposit of metal ion on the hair which will promote, accelerate or catalyze the formation of melanin-like pigments from DHICA or the specified DHICA derivative and, if present, from a dihydroxyindole. Among the preferred metal ions to be present in the metal ion containing compositions employed in this invention the following may be mentioned: Cu⁺², Zn⁺², Mn⁺², Co⁺², Fe⁺², Cr⁺³ Fe⁺³, Mo⁺² and Ti⁺². These may be incorporated in the metal ion composition disclosed herein in the form of salts that are preferably water soluble salts. The anions of said salts again might be quite varied. These include such anions as the sulfate, acetate, nitrate, lactate and citrate anions.

The vehicle of the metal ion containing compositions will usually be an aqueous vehicle in which the metal ions are in solution. The concentration of the metal ions need only be sufficient to complex with hair to promote, accelerate or catalyze the pigment formation from the DHICA. This concentration will be in the range of from 0.0004M to 1.0M with the optimum concentration being about 0.04M.

It may be sometimes advantageous to employ one or more reducing agents to modulate the dyeout color obtained with the DHICA or specified DHICA derivative compositions utilized in this invention. A variety of reducing agents may be used for this purpose. By way of exemplification of suitable reducing agents, mention may be made of the dithionites (e.g. alkali metal dithionites), bisulfites (e.g. alkali metal or alkaline earth meal bisulfites), thioglycollates (e.g. ammonium, alkali metal or alkaline earth metal thioglycollates).

When a reducing agent is used in the practice of the present invention it may be used alone in a carrier (e.g. an aqueous carrier) or as part of the composition containing the metal ions or the DHICA or specified derivative thereof, either alone or in admixture with a dihydroxyindole. In any event, the concentration of the reducing agent in these compositions may vary and generally will be in the range of from 0.01% to 3.0% by weight based on the total weight of the composition in which it is contained the preferred concentrations being from 0.01% to 2% on the same weight basis.

The reducing agent may be applied in any of a number of fashions. Thus it may be applied as an independent step from a composition that contains none of metal ions, DHICA or DHI. In this case, it may proceed or follow the application of the metal ion containing composition. It may also be applied before or after the application of the DHICA/DHI composition. Similarly, when contained in the DHICA/DHI containing composition it may be applied before or after the application of the metal ion composition.

In a preferred aspect of this invention, the reducing agent will constitute part of the metal ion containing composition and will be employed as a pretreatment before the application of the DHICA or specified DHICA derivative composition.

When it is desired to lighten the obtained dyeouts, this can be accomplished by subjecting the colored hair resulting from the above described process to the action of an oxidizing or reducing agent. A variety of such agents are known in the hair dye art which are suitable for the purpose. These are exemplified by such oxidizing agents as H₂O₂, persulfates, or reducing agents such as sulfites.

The oxidizing or reducing agent will generally be applied from a composition containing the same which ordinarily will be an aqueous composition. The concentration of such agents in the lightening composition may vary somewhat but for the most part will be in the range of from 0.001% to 5.00% by weight based on the total weight of the composition in which it is contained.

In carrying out the process of the present invention the hair will be treated with the various compositions in amounts Sufficient to thoroughly wet the hair. The metal ion composition may be applied over a range of time periods which will generally be from 5 to 30 minutes with the optimum treating time being about 10 minutes. This time of application will be about the same regardless of whether the metal ion composition contains a reducing agent. The application of the metal ion composition will take place at about room temperature although somewhat elevated temperatures may also be employed.

The application of the DHICA composition may also take place over a range of time periods with the usual time period being in the range of from 5 to 60 minutes, the optimum time period being about 10 minutes. The DHICA composition will also usually be applied at ambient temperatures but again somewhat elevated temperatures can be employed.

When used separately the reducing composition will be applied over a time range of from 5 to 30 minutes.

Following the application of the treatment compositions employed in the present process the hair will ordinarily be rinsed with water and blow-dried.

The following examples are given to further illustrate this invention. It is understood, however, that this invention is not limited thereto.

A summary of solution preparations is followed by Table I which gives the colors obtainable using said solutions on blended grey hair. Table II summarizes the effects of post-treatment of dyed hair with either alkaline peroxide or persulfate solutions.

### EXAMPLES AND COMPARATIVE EXAMPLES

General methods of preparation of buffers, metal salt solutions, indole dye compositions, reducing agent solutions and peroxide solutions.

### 1. Buffer solutions;

(a) pH 3 - one gram of sodium phosphate (Na H₂PO₄.H₂O) is dissolved in 100ml of water and to this is added 0.85ml of phosphoric acid (H₃PO₄) to give a 0.07m pH 3 phosphate buffer.
(b) pH 5 - one gram of sodium acetate (NaOCOCH₃) and 1.2 ml of acetic acid are mixed into 100 ml of water to give a 0.12M pH 5 acetic acid buffer.
(c) pH 7 - one gram of sodium phosphate (NaH₂PO₄.H₂O) and seventeen hundred milligrams of dibasic phosphate (Na₂HPO₄) are mixed together into 100 ml of water to give a pH 7 0.11M phosphate buffer.
(d) pH 8 - one ml of monoethanolamine (NH₂CH₂CH₂OH) is added to 100 ml of water and 0.2 ml of 6N HCl added to give a 0.1M pH 8.1 buffer.
(e) pH 9 - one ml of monoethanolamine (NH₂CH₂CH₂OH) is added to 100 ml of water to give a 0.1 M pH 9.3 buffer.

### 2. Metal Salt Solutions;

(a) Copper (II) Sulfate - one gram of copper sulfate-pentahydrate (CuSO₄.5H₂O) is dissolved in 100 ml of pH 9 buffer to give 0.04M Cu⁺².
(b) Zinc (II) - 0.5 gm of zinc sulfate heptahydrate (ZnSO₄.7H₂O) is dissolved in 100 ml of pH 8 buffer or pH 5 buffer to give a 0.03M solution of Zn².
(c) Iron (II) - 0.7 gm of ferrous acetate [Fe(OCOCH₃)₂] is dissolved in pH 5 buffer (100 ml) to give a 0.04M Fe⁺² solution.

### 3. Indole dye compositions

(a) DHICA - 0.1 gm of 5,6-dihydroxyindole-2-carboxylic acid (DHICA) is dissolved in 3.0 ml of 95% ethanol and this is added portion-wise to 7.0 ml of a buffer solution to give 1% DHICA at the appropriate pH.
(b) NCH₃DHI/DHICA - 0.5 gm of 5,6-dihydroxy-N-methylindole (NCH₃DHI) and 0.05 gm DHICA are dissolved together in 3.0 ml of 95% ethanol and then this is added portion-wise to pH 9 buffer to give a 0.5% NCH₃DHI/0.5% DHICA (wt/wt) dye composition. Other wt% composition mixtures of NCH₃DHI/DHICA are prepared similarly.
(c) DHI/DHICA - 0.05 gm of DHICA in 3.0 ml of 95% ethanol is added 0.01 gm of 5,6-dihydroxyindole and this solution is added portionwise to 7.0 ml of pH 9 buffer to give a 0.5% DHICA/0.1% DHI dye composition. Other wt% DHICA/DHI compositions are prepared similarly.

### 4. Post-treatment Solutions

(a) 3% H₂O₂ - 50 ml of 20 volume hydrogen peroxide is added to 40 ml of water along with 10 ml monoethanolamine to give a pH 9.2 solution.
(b) 1% Sodium Dithionite - 1.0 gm of sodium dithionite (Na₂S₂O₄) is added to 100 ml of pH 5 buffer to give a 1% solution.
(c) 1% ammoniumthioglycollate - 1.7 ml of a 60% ammonium thioglycollate solution (NH₄CO₂CH₂SH) is added to 98.3 ml of pH 8 buffer to give a 1% solution.

The term C.Ex. when used herein means Comparative Example.

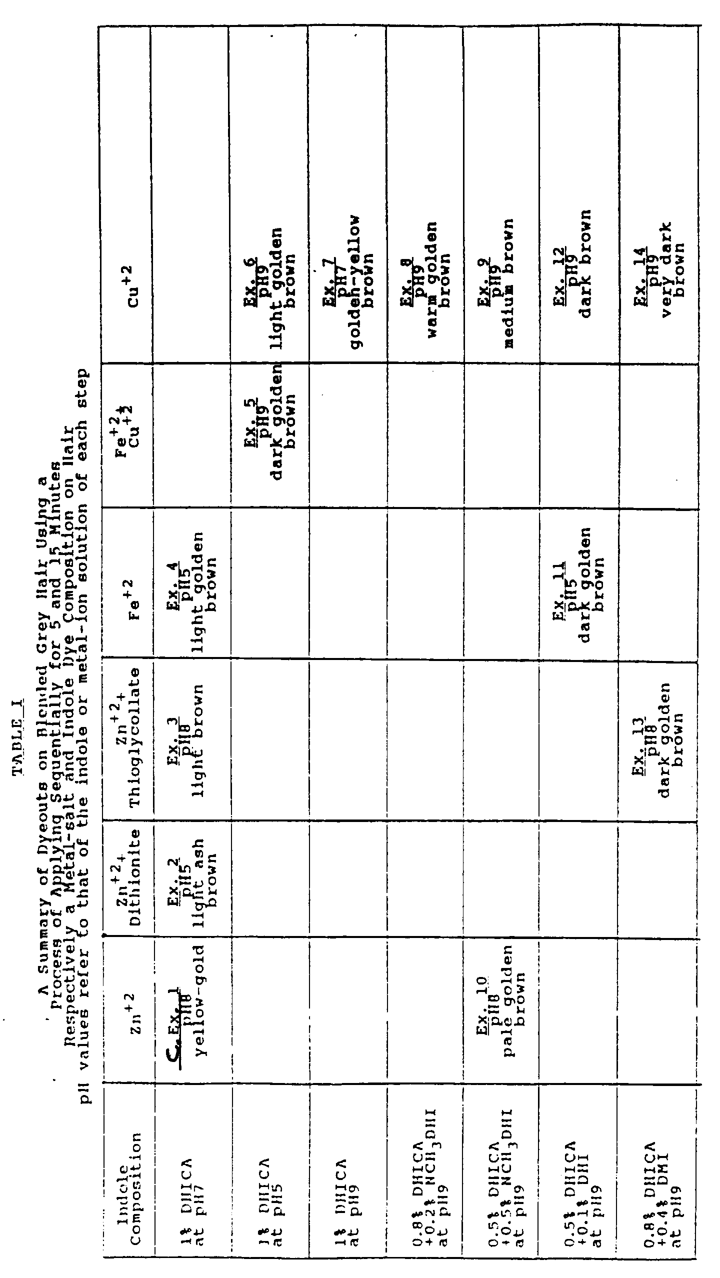

## Claims

1. A process for colouring human hair a brown colour which comprises sequentially treating said hair with a transitional metal ion containing composition, and with a dye composition said dye composition comprising from 0.001% to 10% by weight based on the total weight of said dye composition of a compound of formula I 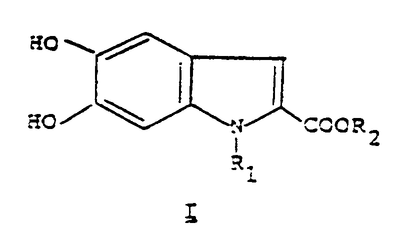
R₁, R₂ = H, alkyl, aryl, or benzyl
the concentration of metal ion in said metal ion containing composition being in the range 0.0004 M to 1.0 M and sufficient to promote the conversion of said compound of formula I to a melanin-like pigment.

2. A process according to claim 1 wherein said compound of formula I is 5,6-dihydroxyindole-2-carboxylic acid.

3. A process according to any preceding claim wherein said metal ion is selected from Cu⁺², Fe⁺², Fe⁺³, Zn⁺², Mn⁺², Co⁺², Mo⁺², Cr⁺³ and Ti⁺².

4. A process according to any preceding claim wherein the dye composition also contains a dihydroxyindole.

5. A process according to claim 4 wherein the dihydroxyindole is a 5,6-dihydroxyindole.

6. A process according to any preceding claim wherein the pH of the hair dye composition is in the range of from 3 to 10.

7. A process according to any preceding claim wherein said metal ion containing composition and said dye composition are applied sequentially in either order.

8. A process according to any preceding claim wherein said hair is also treated with a reducing agent to modulate the color of the hair dye-out.

9. A process according to claim 8 wherein said reducing agent is applied in an independent step.

10. A process according to claim 8 wherein said reducing agent is applied to the hair as part of said metal ion containing composition.

11. A process according to any one of the preceding claims including a further step of treating said hair with an oxidising agent to lighten the dye-out obtained.

## Patentansprüche

1. Verfahren zum Färben von menschlichem Haar mit einer braunen Färbung, in welchem das Haar nacheinander behandelt wird mit einer Übergangsmetallion enthaltenden Zusammensetzung und mit einer Farbstoffzusammensetzung, bezogen auf das Gesamtgewicht der Farbstoffzusammensetzung aufweisend 0,001 bis 10 Gew.-% eine Verbindung der Formel: 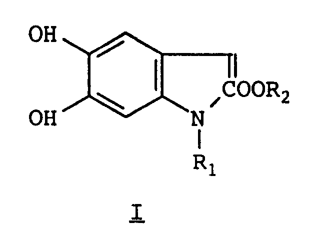
R1, R2 = H, Alkyl, Aryl oder Benzyl
worin die Konzentration des Metallions in der Metallion enthaltenden Zusammensetzung im Bereich von 0,0004 M bis 1,0 M liegt und ausreichend ist, um die Umwandlung der genannten Verbindung der Formel I in ein Melanin-ähnliches Pigment zu fördern.

2. Verfahren nach Anspruch 1, in welchem die Verbindung der Formel I die 5,6-Dihydroxyindol-2-carbonsäure ist.

3. Verfahren nach einem der vorhergehenden Ansprüche, in das Metallion ausgewählt wird aus: Cu⁺², Fe⁺², Fe⁺³, Zn⁺², Mn⁺², Co⁺², Mo⁺², Cr⁺³ und Ti⁺².

4. Verfahren nach einem der vorhergehenden Ansprüche, in welchem die Farbstoffzusammensetzung ferner ein Dihydroxyindol enthält.

5. Verfahren nach Anspruch 4, in welchem das Dihydroxyindol ein 5,6-Dihydroxyindol ist.

6. Verfahren nach einem der vorhergehenden Ansprüche, in dem der pH-Wert der Haarfarbstoffzusammensetzung im Bereich von 3 bis 10 liegt.

7. Verfahren nach einem der vorhergehenden Ansprüche, in dem die Metallion enthaltende Zusammensetzung und die Farbstoffzusammensetzung in beliebiger Reihenfolge nacheinander aufgetragen werden.

8. Verfahren nach einem der vorhergehenden Ansprüche, in dem das Haar zum Modulieren dar Farbe des auf das Haar aufgezogenen Farbstoffes mit einem Reduktionsmittel behandelt wird.

9. Verfahren nach Anspruch 8, in dem das Reduktionsmittel in einem eigenen Schritt aufgetragen wird.

10. Verfahren nach Anspruch 8, in dem das Reduktionsmittel als Teil der Metallion enthaltenden Zusammensetzung auf das Haar aufgetragen wird.

11. Verfahren nach einem der vorhergeheden Ansprüche mit einem weiteren Schritt, in dem zum Aufhellen des aufgezogenen Farbstoffs das Haar mit einem Oxidstionsmittel behandelt wird.

## Revendications

1. Un procédé de coloration de la chevelure humaine en brun qui comprend le traitement séquentiel de ladite chevelure par une composition contenant un ion d'un métal de transition et par une composition colorante, ladite composition colorante comprenant entre 0,001 et 10% en poids par rapport au poids total de ladite composition colorante d'un dérivé de formule (I): 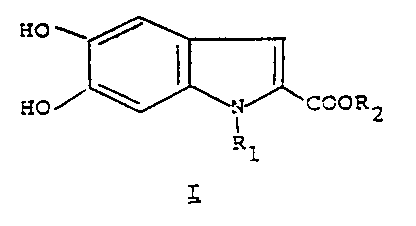
R₁, R₂ = H, un radical alkyle,
aryle ou benzyle
la concentration de l'ion métallique dans ladite composition contenant un ion métallique étant comprise entre 0,0004M et 1,0M et étant suffisante pour entraîner la conversion dudit dérivé de formule (I) en un pigment de type mélanine.

2. Un procédé selon la revendication 1 caractérisé en ce que ledit dérivé de formule I, correspond à de l'acide 5,6-dihydroxyindole-2-carboxylique.

3. Un procédé selon l'une quelconque des revendications précédentes, caractérisé en ce que ledit ion métallique est sélectionné parmi Cu²⁺, Fe²⁺, Fe³⁺, Zn²⁺, Mn²⁺, Co²⁺, Mo²⁺, Cr³⁺, et Ti²⁺.

4. Un procédé selon l'une quelconque des revendications précédentes, caractérisé en ce que la composition colorante contient également du dihydroxyindole.

5. Un procédé selon la revendication 4, caractérisé en ce que le dihydroxyindole correspond à du 5,6-dihydroxyindole.

6. Un procédé selon l'une quelconque des revendications précédentes, caractérisé en ce que le pH de la composition colorant les cheveux est compris entre 3 et 10.

7. Un procédé selon l'une quelconque des revendications précédentes, caractérisé en ce que ladite composition contenant un ion métallique et ladite composition colorante sont appliquées de façon séquentielle dans un ordre quelconque.

8. Un procédé selon l'une quelconque des revendications précédentes, caractérisé en ce que ladite chevelure est également traitée par un agent réducteur afin de modifier la couleur de la coloration des cheveux.

9. Un procédé selon la revendication 8, caractérisé en ce que ledit agent réducteur est appliqué lors d'une étape indépendante.

10. Un procédé selon la revendication 8, caractérisé en ce que ledit agent réducteur est appliqué sur la chevelure en tant que partie de ladite composition contenant l'ion métallique.

11. Un procédé selon l'une quelconque des revendications précédentes, qui inclut une étape supplémentaire de traitement de ladite chevelure avec un agent oxydant pour éclaircir la teinte obtenue.
